# EUROPEAN PATENT APPLICATION

(11) **EP 4 015 625 A1**
(43) Date of publication of application: **22.06.2022**
(21) Application number: 20846905.6
(22) Date of filing: 03.08.2020
(51) Int. Cl.: C12N 5/0783, A61K 35/17, A61P 35/00

(54) **UNIVERSAL CAR-T CELL AND PREPARATION AND USE THEREOF**

(30) Priority: 01.08.2019 CN 201910708725
(71) Applicant: Cellular Biomedicine Group HK Limited, Admiralty, Hong Kong (HK)
(72) Inventor: JI, Yun, Shanghai 201210 (CN); YANG, Weizhi, Shanghai 201210 (CN); CUI, Huijuan, Shanghai 201210 (CN); LUO, Xiaobing, Shanghai 201210 (CN); HUANG, Jiaqi, Shanghai 201210 (CN); ZHU, Shigui, Shanghai 201210 (CN); YAO, Xin, Shanghai 201210 (CN); YAO, Yihong, Shanghai 201210 (CN)
(74) Representative: Cabinet Becker et Associés
(86) International application number: PCT/CN2020/106635
(87) International publication number: WO 2021/018311

(57) **Abstract**

Provided is a non-natural T population, of which the proportion of T memory stem cells satisfies C1≥50%. The T cell population comprises a tumor antigen-targeting universal CAR-T cell. Further provided is a method for preparing the CAR-T cell. The CAR-T cell is higher in amplification rate and better in phenotype, IFN-gamma releasing capacity and target cell killing capability.

## Description

### Technical Field

The present invention relates to the field of biomedicine, and more particularly to a universal CAR-T cell and a preparation and an application thereof.

### Background Art

Immunotherapy, especially adoptive T-cell therapy, has shown strong efficacy and promise in clinical trials for the treatment of hematological malignancies. T cells can be genetically modified to express a chimeric antigen receptor (CAR), which includes an antigen recognition moiety and a T cell activation region. CAR is able to exploit the antigen-binding properties of a monoclonal antibody to redirect T cell specificity and reactivity, and can target a target in an MHC-independent manner. This type of MHC- independent antigen recognition enables CAR-expressing T cells to recognize antigens without the need for antigen processing; a major mechanism of tumor escape is thus avoided. In addition, CAR does not dimerize with an α chain or β chain of endogenous TCR.

Universal CAR-T cells are an important alternative resource for autologous T cells in T cell immunotherapy; the endogenous TRAC and B2M genes of allogeneic T cells are knocked out to prevent graft-versus-host disease and host immune rejection, respectively. These gene-edited cells from allogeneic donors offer cellular immunotherapy opportunities for patients who cannot provide enough autologous T cells, such as infants or patients who have received multiple rounds of chemotherapy.

At present, there is no universal CAR-T cell product approved on the market in China and abroad. In addition, there are still many deficiencies in the research on universal CAR-T cells in the art. Thus, there is an urgent need in this field to develop new universal CAR-T cell products with desired killing effect and high safety.

### Summary of the Invention

On object of the present invention is to provide Tscm-enriched universal CAR-T cells, as well as a preparation and an application thereof.

In a first aspect of the present invention, a non-naturally occurring T cell population is provided. The proportion C1 of T memory stem cells (stem cell-like memory T cells, Tscm) in the T cell population is ≥50%, calculated based on the total number of T cells in the T cell population.

In another preferred embodiment, the C1≥60%, preferably C1≥70%, and more preferably C1≥75%.

In another preferred embodiment, the C1 is 50-85%, and preferably 60-80%.

In another preferred embodiment, the T cells include CAR-T cells.

In another preferred embodiment, the CAR-T cells include autologous or allogeneic CAR-T cells.

In another preferred embodiment, the CAR-T cells include universal CAR-T cells.

In another preferred embodiment, in the CAR-T cell, an immune-related gene selected from the following group is knocked out or down-regulated: TRAC gene, B2M gene, or a combination thereof.

In another preferred embodiment, when the T memory stem cells (Tscm) are CAR-T cells, the proportion C1 of the T memory stem cells (Tscm) is greater than or equal to 50%, calculated based on the total number of CAR-T cells in the T cell population.

In another preferred embodiment, the C1≥60%, preferably C1≥70%, and more preferably C1≥75%.

In another preferred embodiment, the C1 is 50-85%, and preferably 60-80%.

In another preferred embodiment, the T memory stem cells have a CCR7⁺ phenotype.

In another preferred embodiment, in the T cell population, the content of T cells C3 ≥ 30%, preferably ≥50%, more preferably ≥70%, and even more preferably ≥80%, ≥90%, or ≥95%, calculated based on the total number of cells in the cell population.

In another preferred embodiment, C3 is 30-100%, more preferably 70-99.9%, and even more preferably 80-99%.

In another preferred embodiment, the T memory stem cells include CCR7⁺CD45RA+ T cells.

In another preferred embodiment, in the T cell population, the proportion C2 of expressing a biomarker CCR7 is ≥50%, preferably ≥60%, and more preferably ≥70%.

In another preferred embodiment, the activity intensity Q1 of the CAR-T cells (taking the mean fluorescence intensity MFI as an example) ≥500, preferably ≥1000, more preferably ≥3000, and even more preferably ≥4000 (for example, 1000-5000).

In another preferred embodiment, the T cell population is expanded or not expanded in vitro.

In another preferred embodiment, the in vitro amplification is to perform cell amplification in the presence of IL15, IL7 and IL21 (commonly referred to as Tscm amplification factors) in the culture system under conditions suitable for cell culture.

In another preferred embodiment, in the culture system, the concentration of the Tscm amplification factor includes:
the concentration of IL15 is 1-200 ng/ml, preferably 3-100 ng/ml, and more preferably 5-20 ng/ml;
the concentration of IL7 is 0.5-50 ng/ml, preferably 1-20 ng/ml, and more preferably 3-10 ng/ml; and/or
the concentration of IL21 is 1-100 ng/ml, preferably 3-50 ng/ml, and more preferably 5-20 ng/ml.

In another preferred embodiment, the culture system includes a serum culture system and a serum-free culture system.

In another preferred embodiment, the cell amplification time is 0.1-30 days, preferably 0.5-25 days, more preferably 1-20 days, and even most preferably 2-15 days, or 3-15 days, or 5-15 days.

In another preferred embodiment, the CAR-T cells are universal CAR-T cells targeting tumor antigens.

In another preferred embodiment, the tumor antigen is selected from the group consisting of: TSHR, CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM , B7H3, KIT, IL-13Ra2, mesothelin, IL-11Ra, PSCA, PRSS21, VEGFR2, LewisY, CD24, PDGFR-β, SSEA-4, CD20, folate receptor α, ERBB2(Her2/neu), MUC1 , EGFR, NCAM, Prostase, PAP, ELF2M, Ephrin B2, IGF-I receptor, CAIX, LMP2, gp100, bcr-abl, tyrosinase, EphA2, fucosyl GM1, sLe, GM3, TGS5 , HMWMAA, o-acetyl-GD2, folate receptor β, TEM1/CD248, TEM7R, CLDN6, GPRC5D, CXORF61, CD97, CD179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos-associated antigen 1, p53, p53 mutant, prostein, survivin and telomerase, PCTA-1/Galectin 8, MelanA/MARTI, Ras mutant, hTERT, Sarcoma translocation breakpoint, ML-IAP, ERG (TMPRSS2ETS fusion gene), NA17, PAX3, androgen receptor, cyclin B1, MYCN, RhoC, TRP-2, CYP1B1, BORIS, SART3, PAX5, OY-TES1 , LCK, AKAP-4, SSX2, RAGE-1, human telomerase reverse transcriptase, RU1, RU2, intestinal carboxylesterase, mut hsp70-2, CD79a, CD79b, CD72, LAIR1, FCAR, LILRA2, CD300LF, CLEC12A , BST2, EMR2, LY75, GPC3, FCRL5, IGLL1, and a combination thereof.

In another preferred embodiment, the CAR-T cell expresses a chimeric antigen receptor, and the structure of the chimeric antigen receptor is shown in the following formula I:

L-scFv-H-TM-C-CD3ζ (I)

In the formula,
each "-" is independently a linking peptide or peptide bond;
L is an optional signal peptide sequence;
scFv is an antibody single chain variable region sequence targeting a tumor antigen;
H is an optional hinge region;
TM is a transmembrane domain;
C is no or costimulatory signal molecule; and
CD3ζ is a cytoplasmic signaling sequence derived from CD3ζ.

In another preferred embodiment, the CAR-T cells are universal CAR-T cells targeting BCMA.

In another preferred embodiment, the scFv targets BCMA.

In another preferred embodiment, the L is a signal peptide of a protein selected from the group consisting of CD8, GM-CSF, CD4, CD137, and a combination thereof.

In another preferred embodiment, the H is a hinge region of a protein selected from the group consisting of CD8, CD28, CD137, and a combination thereof.

In another preferred embodiment, the TM is a transmembrane region of a protein selected from the group consisting of CD28, CD3 epsilon, CD45, CD4, CD5, CD8, CD9, CD16, CD22, CD33, CD37, CD64, CD80, CD86, CD134, CD137, CD154, and a combination thereof.

In another preferred embodiment, the C is a costimulatory signal molecule of a protein selected from the group consisting of OX40, CD2, CD7, CD27, CD28, CD30, CD40, CD70, CD134, 4-1BB (CD137), PD1, Dap10, CDS, ICAM-1, LFA-1 (CD11a/CD18), ICOS (CD278), NKG2D, GITR, TLR2, and a combination thereof.

In another preferred embodiment, C includes a costimulatory signal molecule derived from 4-1BB, and/or a costimulatory signal molecule derived from CD28.

In another preferred embodiment, the T cells are derived from human or non-human mammals.

In another preferred embodiment, the T cell population is prepared by the method described in the third aspect of the present invention.

In a second aspect of the present invention, a cell preparation is provided, the cell preparation includes the non-naturally occurring T cell population according to the first aspect of the present invention, and a pharmaceutically acceptable carrier, diluent or excipient.

In a third aspect of the present invention, a method for preparing CAR-T cells is provided, which includes the following steps:
(a) providing isolated T cells;
(b) performing amplification culture on the isolated T cells in the presence of IL15, IL7 and IL21 to obtain cultured T cells; and
(c) performing engineering modification on the cultured T cells to prepare the CAR-T cells.

In another preferred embodiment, in step (a), the isolated T cells are selected from the group consisting of naive T cells (Tn cells), a Tn cell-rich cell population, total T cells, and a combination thereof.

In another preferred embodiment, the isolated T cells are naive T cells (Tn cells) or a Tn cell-rich cell population.

In another preferred embodiment, the T cells include autologous or allogeneic T cells.

In another preferred embodiment, in step (b), when the isolated T cells are cultured for amplification, the concentration of IL15 in the culture system is 1-200 ng/ml, preferably 3-100 ng/ml, and more preferably 5-20 ng/ml; the concentration of IL7 is 0.5-50 ng/ml, preferably 1-20 ng/ml, and more preferably 3-10 ng/ml; and the concentration of IL21 is 1-100 ng/ml, preferably 3-50 ng/ml, and more preferably 5-20ng/ml.

In another preferred embodiment, the method is used to prepare universal CAR-T cells.

In another preferred embodiment, the CAR-T cells target the tumor antigen BCMA.

In another preferred embodiment, in step (a), the T cells are isolated from human peripheral blood, and preferably peripheral blood mononuclear cells.

In another preferred embodiment, the isolated T cells include total T cells and naive T cells (Tn cells).

In another preferred embodiment, the naive T cells are CCR7⁺CD45RA⁺ T cells.

In another preferred embodiment, in step (b), the amplification culture is performed in an AIM-V culture medium.

In another preferred embodiment, in step (b), the culture system further includes FBS, Glutamax, Pen/Strip, or a combination thereof; and preferably, a concentration of the FBS is 5-20%.

In another preferred embodiment, in step (c), the medication includes T cell activation, T cell nucleofection, and lentiviral transduction.

In another preferred embodiment, in step (c), the medication includes the following steps:
(1) activating the cultured T cells with CD3/CD28 magnetic beads, and then removing the magnetic beads to obtain the activated T cells;
(2) performing nucleofection on the activated T cells using a CRISPR-Cas9 system to knock out a TRAC gene and/or B2M gene of the T cells, thereby obtaining nuclear transfected T cells; and
(3) transducing the nuclear transfected T cells with a viral vector containing a CAR expression cassette to prepare CAR-T cells.

In a fourth aspect of the present invention, a method for preparing CAR-T cells is provided, which includes the following steps:
(i) providing isolated Tn cells,
(ii) optionally, performing amplification culture on the isolated T cells in the presence of IL15, IL7 and IL21 to obtain cultured T cells; and
(iii) modifying the T cells to prepare CAR-T cells.

In a fifth aspect of the present invention, a use of the T cell population (especially CAR-T cells) set forth in the first aspect of the present invention, or the cell preparation set forth in the second aspect of the present invention is provided for the preparation of a medicament for preventing and/or treating cancer or tumors.

In another preferred embodiment, the tumor is selected from the group consisting of hematological tumors, solid tumors, and a combination thereof.

In another preferred embodiment, the hematological tumors are selected from the group consisting of acute myeloid leukemia (AML), multiple myeloma (MM), chronic lymphocytic leukemia (CLL), acute lymphoblastic leukemia (ALL), diffuse B-cell lymphoma (DLBCL), and a combination thereof.

In another preferred embodiment, the solid tumor is selected from the group consisting of: gastric cancer, gastric cancer peritoneal metastasis, liver cancer, leukemia, kidney tumor, lung cancer, small intestine cancer, bone cancer, prostate cancer, colorectal cancer, breast cancer, colorectal cancer, cervical cancer, ovarian cancer, lymphoma, nasopharyngeal cancer, adrenal tumor, bladder tumor, non-small cell lung cancer (NSCLC), glioma, endometrial cancer, testicular cancer, colorectal cancer, urinary tract tumor, thyroid cancer, and a combination thereof.

In another preferred embodiment, the solid tumor is selected from the group consisting of: ovarian cancer, mesothelioma, lung cancer, pancreatic cancer, breast cancer, liver cancer, endometrial cancer, and a combination thereof.

In a sixth aspect of the present invention, a use of the T cell population set forth in the first aspect of the present invention, or the cell preparation set forth in the second aspect of the present invention is provided for preventing and/or treating cancer or tumors.

In a seventh aspect of the present invention, a method for treating a disease is provided, which includes: administering an appropriate amount of the T cell population set forth in the first aspect of the present invention or the cells set forth in the second aspect of the present invention to a subject in need thereof.

In another preferred embodiment, the disease is cancer or tumors.

It should be understood that, within the scope of the present invention, the above-mentioned technical features of the present invention and the technical features specifically described in the following (for example, in the embodiments) can be combined with each other, thereby forming new or preferred technical solutions. Due to space limitations, it is not repeated herein.

### Brief Description of the Drawings

Fig. 1 shows the gene knockout and purification rates of TRAC and B2M in universal T cells detected by flow staining.
Fig. 2 shows the transduction rate of BCMA CAR in universal T cells detected by flow staining.
Fig. 3 shows ELISA detection of IFN-γ release after co-culture of universal CAR-T cells with BCMA-expressing target cells.
Fig. 4 shows the phenotype and purity of naive T cells isolated from PBMCs by flow cytometry.
Fig. 5 shows the proliferation rates of three BCMA CAR-T cells.
Fig. 6 shows the ratio of various T cell subtypes and the expression of CCR7 in the three types of BCMA CAR-T cells.
Fig. 7 shows the CAR transfection efficiencies of three BCMA CAR-T cells detected by flow cytometry.
Fig. 8 shows the release of IFN-γ after co-culture of three types of BCMA CAR-T cells and BCMA-expressing target cells.
Fig. 9 shows the killing rates of three types of BCMA CAR-T cells against BCMA-expressing target cells.
Fig. 10 shows the enrichment and cell growth of Tscm cells in T cells cultured under different conditions.
Fig. 11 shows the BCMA CAR transduction rates of T cells cultured under different conditions.
Fig. 12 shows IFN-γ release following BCMA CAR transduction of T cells cultured under different conditions.
Fig. 13 shows the enrichment of CCR7⁺CD45RA⁺ Tscm cells of three types of BCMA CAR-T cells.

For the labels used in the accompanying drawings of the present invention, their specific meanings are as follows:
mock: T cells without Cas9 RNP nucleofection; mock+ CAR: T cells transduced with BCMA CAR without Cas9 RNP nucleofection; CAR: T cells transduced by BCMA CAR; double KO: T cells with double knockout of TRAC and B2M after Cas9 RNP nucleofection; Double KO + CAR: T cells transduced by the above Double KO cells with BCMA CAR; BCMA CAR: traditional autologous CAR-T cells transduced with BCMA CAR; 1°U CAR: the first generation of universal T cells transduced by BCMA CAR; and 2°U CAR: the second generation of universal T cells transduced with BCMA CAR.

### Description of the Embodiments

After extensive and in-depth studies, the inventors unexpectedly have found for the first time that when T cells are cultured under conditions that favor T memory stem cell (Tscm) amplification, the prepared Tscm-enriched universal BCMA CAR-T cells exhibit a better amplification rate, phenotype, IFN-γ releasing ability and killing ability for target cells as compared with the first generation of cells. Based on the Tscm-enriched T cells of the present invention (especially the second-generation universal CAR-T cells), highly active universal CAR-T cells for different targets can be prepared. Thus, the present invention has been completed on this basis.

Specifically, in order to further improve the efficacy of the universal BCMA CAR-T cells,
T cells have been cultured under conditions that favor the amplification of T memory stem cells (Tscm), such that a new second generation of Tscm-enriched universal CAR-T cells is prepared. The CAR-T cells are much better than the first generation of universal CAR-T cells in terms of proliferation, phenotype, and killing of target cells. This indicates that the second generation of the universal CAR-T cells is further optimized universal CAR-T products.

Typically, a Cas9 RNP complex can be delivered into T cells by means of nucleofection to simultaneously knock out endogenous TRAC and B2M genes. After purification, high-purity TRAC and B2M double knockout universal T cells can be prepared. The T cells are then introduced with the BCMA CAR expression by means of lentiviral transduction so as to generate the first generation of universal BCAM CAR-T cells. These CAR-T cells can effectively kill target cells. Their activity is similar to that of autologous CAR-T cells without gene editing.

### Terminology

To make the present disclosure readily understandable, certain terms are firstly defined. As used in the present application, unless otherwise explicitly stated herein, each of the following terms has the meaning provided below. Additional definitions are set forth throughout the present application.

The term "about" may refer to a value or composition within an acceptable error range of a particular value or composition as determined by a person of ordinary skill in the art. It will depend in part on how the value or composition is measured or determined.

The term "administration" refers to the physical introduction of a product of the present invention into a subject using any one of a variety of methods and delivery systems known to a person skilled in the art, which include intravenous, intramuscular, subcutaneous, intraperitoneal, spinal, or other parenteral routes of administration, for example, by injection or infusion.

The term "antibody" (Ab) includes, but is not limited to, immunoglobulins. It can specifically bind antigen and includes at least two heavy (H) chains and two light (L) chains interconnected by disulfide bonds, or antigen a binding portion thereof. Each H chain includes a heavy chain variable region (abbreviated herein as VH) and a heavy chain constant region. The heavy chain constant region contains three constant domains, CH1, CH2 and CH3. Each light chain includes a light chain variable region (abbreviated herein as VL) and a light chain constant region. The light chain constant region contains one constant domain, CL. The VH and VL regions can be further subdivided into hypervariable regions which may be referred to as complementarity determining regions (CDRs), which are interspersed with more conserved regions called framework regions (FRs). Each VH or VL contains three CDRs and four FRs, which are arranged from the amino terminus to the carboxyl terminus in the following order: FR1, CDR1, FR2, CDR2, FR3, CDR3, FR4. The variable regions in the heavy chains and light chains contain binding domains that interact with an antigen.

It should be understood that the names of amino acids in the present application are identified by the internationally accepted single English letters, and the corresponding three-letter abbreviations of these amino acid names are as follows: Ala (A), Arg (R), Asn (N), Asp (D), Cys (C), Gln (Q), Glu (E), Gly (G), His (H), Ile (I), Leu (L), Lys (K), Met (M), Phe (F), Pro (P), Ser (S), Thr (T), Trp (W), Tyr (Y), and Val (V).

### Universal CAR-T cells

Universal T cells are an important alternative resource for autologous T cells in T cell immunotherapy. Endogenous TRAC and B2M genes of allogeneic T cells can be knocked out in order to prevent graft-versus-host disease and host immune rejection, respectively. These gene-edited cells from allogeneic donors can offer cellular immunotherapy opportunities for patients who cannot provide enough autologous T cells, such as infants, and patients who have received multiple rounds of chemotherapy. These cells can be further modified by lentiviral vectors encoding CAR or TCR to generate allogeneic CAR/TCR T cells.

In the present invention, a Cas9 RNP complex has been delivered into T cells by means of nucleofection to simultaneously knock out endogenous TRAC and B2M genes. In addition, BCMA CAR expression is introduced using lentiviral transduction to generate ready-made BCAM CAR-T cells. These CAR-T cells can effectively kill target cells. Their activity is similar to that of autologous BCMA CAR-T cells without gene editing.

In order to further enhance the activity of the universal CAR-T cells, gene editing has been performed from the allogeneic naive T cells (Tn). In addition, special culture conditions that are conducive to the amplification of T memory stem cells (Tscm) are employed to produce the Tscm-enriched second generation of universal BCMA CAR-T cells. These CAR-T cells are significantly superior to the traditional first generation of universal CAR-T cells in terms of amplification, phenotype, IFN-γ release ability, and killing of target cells.

### Tn cells

Tn cells, i.e., naive T cells (Tn), also known as unprimed T cells. They mature in the thymus and then migrate to the peripheral lymphoid tissues, where they are relatively quiescent before exposure to antigenic stimulation.

### Tscm cells

Tscm cells represent T memory stem cells, also known as stem cell memory T cells (Tscm).

### BCMA

BCMA is a B cell maturation antigen, also known as CD269 or TNFRSF17. It is a member of the tumor necrosis factor receptor superfamily. Its ligands are B cell activating factor (BAFF) and proliferation-inducing ligand (APRIL).

Binding of BCMA to BAFF and APRIL triggers NF- κB activation, and induce the upregulation of certain anti-apoptotic Bcl-2 members such as Bcl-xL, or Bcl-2 and Mcl-1. The interaction between BCMA and its ligands regulates humoral immunity from different aspects and the growth and differentiation of B cells so as to maintain the stable balance of the human body environment.

BCMA expression is restricted to B cell lineages. It is expressed on plasmablasts, plasma cells and a subset of mature B cells. It is increased upon differentiation of terminal B cells and is not expressed in most B cells, such as naive B cells, memory B cells and B cell germinal centers and other organs. The expression of BCMA has been reported to be important for long-lived, sessile plasma cells in the bone marrow. Therefore, BCMA-deficient mice have reduced plasma cells in the bone marrow while plasma cell levels in the spleen are unaffected. Mature B cells differentiate normally into plasma cells in BCMA knockout mice. The BCMA knockout mice appear normal and healthy, and have normal numbers of B cells, but their plasma cells cannot survive over a long term.

BCMA is also highly expressed in malignant plasma cells, such as in multiple myeloma and plasma cell leukemia. BCMA has also been detected in HRS cells from Hodgkin lymphoma patients. In the United States, hematological malignancies account for about 10% of all malignant tumors, and myeloma accounts for 15% of all hematological malignancies. It has been reported in the literature that the expression of BCMA is associated with disease progression in multiple myeloma. The BCMA gene is highly expressed in myeloma samples, but the expression is very low in chronic lymphocytic leukemia, acute lymphocytic leukemia, and acute T-cell lymphocytic leukemia. B-cell lymphomas increase markedly in mouse models overexpressing the BCMA ligands BAFF and APRIL. Ligands that bind BCMA have been shown to regulate the growth and survival of BCMA-expressing multiple myeloma cells. Binding of BCMA to BAFF and APRIL allows malignant plasma cells to survive. Thus, depletion of BCMA-expressing tumor cells and disruption of the interaction between BCMA ligands and receptors can improve treatment outcomes for multiple myeloma or other BCMA-positive B-lineage malignant lymphomas.

### Chimeric Antigen Receptors (CARs)

The CAR in the present invention may include any type of antigen binding domain targeting tumor antigens.

In the present invention, the chimeric antigen receptor (CAR) includes an extracellular domain, a transmembrane domain, and an intracellular domain. The extracellular domain includes target-specific binding elements (also referred to as antigen binding domains). The intracellular domain includes a costimulatory signaling region and a ζ chain moiety. The costimulatory signaling region herein refers to a portion of an intracellular domain that includes a costimulatory molecule(s). The costimulatory molecule is a cell surface molecule(s) that is required for an efficient lymphocyte response to an antigen, rather an antigen receptor or a ligand.

A linker can be incorporated between the extracellular domain and the transmembrane domain of the CAR, or between the cytoplasmic domain and the transmembrane domain of the CAR. As used herein, the term "linker" generally refers to any oligopeptide or polypeptide that functions to link the transmembrane domain to the extracellular or cytoplasmic domain of a polypeptide chain. The linker may include 0-300 amino acids, preferably 2 to 100 amino acids, and most preferably 3 to 50 amino acids.

In a preferred embodiment of the present invention, the extracellular domain of the CAR provided by the present invention includes an antigen-binding domain targeting BCMA. The CAR of the present invention, when expressed in T cells, is capable of antigen recognition based on the antigen binding specificity. When it binds to its cognate antigen, it affects tumor cells so that the tumor cells do not grow, are caused to die, or are otherwise affected. This can lead to the reduction or elimination of a patient's tumor burden. The antigen binding domain is preferably fused to an intracellular domain from one or more of the costimulatory molecules and the ζ chain. Preferably, the antigen binding domain is fused to the intracellular domain formed by the combination with the 4-1BB signaling domain and the CD3ζ signaling domain.

As used herein, an "antigen binding domain" and a "single chain antibody fragment" both refer to a Fab fragment, a Fab' fragment, an F(ab')₂ fragment, or a single Fv fragment having the antigen binding activity. An Fv antibody contains antibody heavy chain variable regions, light chain variable regions, but no constant regions; it is the smallest antibody fragment with all antigen-binding sites. Typically, an Fv antibody also contains a polypeptide linker between the VH and VL domains, and is capable of forming the structure required for antigen binding. The antigen binding domain is usually an scFv (single chain variable fragment). The size of scFv is generally 1/6 of that of a complete antibody. A single chain antibody is preferably an amino acid chain sequence encoded by one nucleotide chain. As a preferred mode of the present invention, the scFv may include an antibody that specifically recognizes BCMA, and preferably a single chain antibody.

With regard to the hinge region and the transmembrane region (transmembrane domain), the CAR can be designed to include a transmembrane domain fused to an extracellular domain of the CAR. In one embodiment, the transmembrane domain is naturally associated with one of the domains in the CAR. In some examples, the transmembrane domain may be selected, or modified by amino acid substitutions, to avoid binding such domains to transmembrane domains of the same or different surface membrane proteins, thereby minimizing interaction with other members of the receptor complex.

The intracellular domain in the CAR of the present invention may include the signaling domain of 4-IBB and the signaling domain of CD3ζ.

### Gene silencing methods

At present, the commonly used gene silencing methods include CRISPR/Cas9, RNA interference technology, TALENs (transcription activator-like (TAL) effector nucleases), and Zinc finger nucleases (ZFNs); among them, CRISPR/Cas9 has the best application prospect and effect.

The CRISPR (clustered regularly interspersed short palindromic repeats)/Cas (CRISPR-associated) system is a natural immune system unique to prokaryotes. It is used against virus or foreign plasmids. The Type II CRISPR/Cas system has been successfully applied in many eukaryotes and prokaryotes as a tool for direct RNA-mediated genome editing. The development of the CRISPR/Cas9 system has revolutionized the ability to edit DNA sequences and regulate the expression levels of target genes, thereby providing a powerful tool for precise genome editing of organisms. The simplified CRISPR/Cas9 system is composed of two parts: a Cas9 protein and an sgRNA. The mechanism of action is that the sgRNA forms a Cas9-sgRNA complex with Cas9 protein through its Cas9 handle; a base complementary pairing sequence region of the sgRNA in the Cas9-sgRNA complex is paired with a target sequence of a target gene based on the base complementary pairing principle; Cas9 then utilizes its own endonuclease activity to cleave the target DNA sequence. Compared with the traditional genome editing techniques, the CRISPR/Cas9 system has several distinct advantages: ease of use, simplicity, low cost, programmability, and the ability to edit multiple genes simultaneously.

### Vectors

Nucleic acid sequences encoding the desired molecules can be obtained using recombinant methods known in the art, such as, for example, screening a library from cells expressing the gene, obtaining a gene from a vector known to contain the gene, or direct isolating a gene from cells and tissues containing the gene using standard techniques. Alternatively, the gene of interest can be produced synthetically.

The present invention also provides vectors into which the expression cassettes of the present invention are inserted. Vectors derived from retroviruses such as lentiviruses are suitable tools to achieve long-term gene transfer because they allow long-term, stable integration of the transgene and the proliferation thereof in daughter cells. Lentiviral vectors have advantages over vectors derived from oncogenic retroviruses such as murine leukemia virus, because they can transduce non-proliferating cells, such as hepatocytes. They also have the advantage of low immunogenicity.

In brief overview, an expression cassette or nucleic acid sequence of the present invention is typically operably linked to a promoter and incorporated into an expression vector. The vector is suitable for replication and integration in eukaryotic cells. Typical cloning vectors contain transcriptional and translational terminators, initial sequences and promoters that can be used to regulate the expression of the desired nucleic acid sequences. The expression constructs of the present invention can also be used in nucleic acid immunization and gene therapy based on standard gene delivery protocols. Methods of gene delivery are known in the art. Reference may be made to the following documents: for example, US Patent Nos. 5,399,346, 5,580,859, 5,589,466, which are hereby incorporated by reference in their entirety. In another embodiment, the present invention provides gene therapy vectors.

The nucleic acid can be cloned into various types of vectors. For example, the nucleic acid can be cloned into vectors including, but not limited to, plasmids, phagemids, phage derivatives, animal viruses, and cosmids. Particular vectors of interest herein include expression vectors, replication vectors, probe generation vectors, and sequencing vectors.

Moreover, expression vectors can be provided to cells in the form of viral vectors. The viral vector technology is well known in the art, and has been described in, for example, Sambrook et al. (2001, Molecular Cloning: A Laboratory Manual, Cold Spring Harbor Laboratory, New York) and other virology and molecular biology manuals. Viruses that can be used as vectors include, but are not limited to, retroviruses, adenoviruses, adeno-associated viruses, herpesviruses, and lentiviruses. In general, a suitable vector contains an origin of replication functional in at least one organism, a promoter sequence, convenient restriction enzyme sites, and one or more selectable markers (e.g., WO01/96584; WO01/29058; and US Patent No. 6,326,193).

A number of virus-based systems have been developed for gene transfer into mammalian cells. For example, retroviruses provide a convenient platform for gene delivery systems. A selected gene can be inserted into a vector and packaged into a retroviral particle using techniques known in the art. A recombinant virus can then be isolated and delivered to subject cells in vivo or in vitro. There are many retroviral systems known in the art. In some embodiments, adenoviral vectors are used. There are many adenoviral vectors known in the art. In one embodiment, lentiviral vectors are used.

Additional promoter elements, such as enhancers, can regulate the frequency of transcription initiation. Typically, they are located in a region of 30-110 bp upstream of the initiation site, although it has recently been shown that many promoters also contain functional elements downstream of the initiation site. The spacing between promoter elements is often flexible so that promoter function can be maintained when elements are inverted or moved relative to one another. In a thymidine kinase (tk) promoter, the spacing between promoter elements can be increased by 50 bp before activity begins to decline. Depending on the promoter, individual elements may act cooperatively or independently to initiate the transcription.

An example of a suitable promoter is the immediate early cytomegalovirus (CMV) promoter sequence. This promoter sequence is a strong constitutive promoter sequence capable of driving high-level expression of any polynucleotide sequence operably linked thereto. Another example of a suitable promoter is elongation growth factor-la (EF-1α). However, other constitutive promoter sequences may also be used herein, which include, but are not limited to, the simian virus 40 (SV40) early promoter, mouse mammary tumor virus (MMTV), human immunodeficiency virus (HIV) long terminal repeat (LTR) promoter, MoMuLV promoter, avian leukemia virus promoter, Epstein-Barr virus immediate early promoter, Ruse sarcoma virus promoter, as well as human gene promoters, such as, but not limited to, the actin promoter, myosin promoter, heme promoter and creatine kinase promoter. Furthermore, the present invention is not limited to the use of constitutive promoters. Inducible promoters are also considered as part of the present invention. The use of inducible promoters provides a molecular switch, which can turn on the expression of a polynucleotide sequence operably linked to an inducible promoter when such expression is desired, or turn off the expression when such an expression is not desired. Examples of inducible promoters include, but are not limited to, metallothionein promoters, glucocorticoid promoters, progesterone promoters, and tetracycline promoters.

To assess the expression of a CAR polypeptide or a portion thereof, the expression vectors introduced into cells may also contain either or both of a selectable marker gene and a reporter gene in order to facilitate the identification and selection of expressing cells from a population of cells sought to be transfected or infected by the viral vector. In other aspects, the selectable marker may be carried on a single piece of DNA and used in co-transfection procedures. Both the selectable marker and the reporter gene can be flanked by appropriate regulatory sequences to enable the expression thereof in a host cell. Useful selectable markers include, for example, antibiotic resistance genes, such as neo and the like.

Reporter genes are used to identify potentially transfected cells and to evaluate the functionality of regulatory sequences. Typically, reporter genes are the following genes: the genes that are not present in or expressed by the recipient organism or tissue, and encode polypeptides; in addition, the expression of the polypeptides is clearly indicated by some readily detectable properties such as enzymatic activity. After a DNA is introduced into recipient cells, the expression of the reporter gene can be measured at an appropriate time. Suitable reporter genes include genes encoding luciferase, beta-galactosidase, chloramphenicol acetyltransferase, secreted alkaline phosphatase, and green fluorescent protein (for example, Ui-Tei, et al., 2000 FEBS Letters 479:79-82). Suitable expression systems are well known and can be prepared using known techniques, or obtained commercially. Typically, constructs with a minimum of 5 flanking regions showing the highest levels of reporter gene expression are identified as promoters. The promoter regions can be linked to reporter genes and used to assess the ability of an agent to modulate promoter-driven transcription.

Methods for introducing and expressing genes into cells are known in the art. In the context of an expression vector, the vector can be readily introduced into a host cell by any method known in the art, for example, mammalian, bacterial, yeast or insect cells. For example, an expression vector can be transferred into a host cell by physical, chemical or biological means.

Physical methods of introducing polynucleotides into host cells include calcium phosphate precipitation, lipofection, particle bombardment, microinjection, electroporation, and the like. Methods of producing cells containing vectors and/or exogenous nucleic acids are well known in the art. Reference may be made to, for example, Sambrook et al. (2001, Molecular Cloning: A Laboratory Manual, Cold Spring Harbor Laboratory, New York). The preferred method of introducing polynucleotides into host cells is calcium phosphate transfection.

Biological methods for introducing polynucleotides of interest into host cells include the use of DNA and RNA vectors. Viral vectors, especially retroviral vectors, have become the most widely used method of inserting genes into mammalian cells, such as human cells. Other viral vectors may be derived from lentiviruses, poxviruses, herpes simplex virus I, adenoviruses, adeno-associated viruses, and the like. Reference may be made to, for example, US Patent Nos. 5,350,674 and 5,585,362.

Chemical means of introducing polynucleotides into host cells include colloidal dispersion systems, such as macromolecular complexes, nanocapsules, microspheres, beads; and lipid-based systems, including oil-in-water emulsions, micelles, mixed micelles, and liposomes. Exemplary colloidal systems for use as in vitro and in vivo delivery vehicles are liposomes (for example, artificial membrane vesicles).

In the case where non-viral delivery systems are used, exemplary delivery vehicles are liposomes. The use of lipid formulations may be considered to introduce nucleic acids into host cells (in vitro, ex vivo, or in vivo). In another aspect, the nucleic acids can be associated with lipid. Nucleic acids associated with lipids can be encapsulated into the aqueous interior of liposomes, interspersed within the lipid bilayer of liposomes, attached to liposomes via a linker molecule associated with both the liposomes and the oligonucleotide, entrapped in liposomes, complexed with liposomes, dispersed in lipid-containing solutions, mixed with lipids, associated with lipids, contained in lipids as certain suspensions, contained in micelles or complexed with micelles, or otherwise associated with lipids. The lipid, lipid/DNA or lipid/expression vector associated with a composition is not limited to any particular structure in a solution. For example, they may exist in bilayer structures, as micelles, or have a "collapsed" structure. They can also simply be dispersed in a solution, possibly forming aggregates that are not uniform in size or shape. Lipids are fatty substances. They can be certain naturally occurring or synthetic lipids. For example, lipids may include fat droplets. They may occur naturally in the cytoplasm and in such compounds including long chain aliphatic hydrocarbons and derivatives thereof, such as fatty acids, alcohols, amines, amino alcohols and aldehydes.

In a preferred embodiment of the present invention, the vector is a lentiviral vector.

### Preparations

The present invention provides a CAR-T cell containing the first aspect of the present invention, and a pharmaceutically acceptable carrier, diluent or excipient. In one embodiment, the preparation is a liquid preparation. Preferably, the preparation is an injection. Preferably, the concentration of the CAR-T cells in the preparation is 1 × 10³-1 × 10⁸ cells/ml, and more preferably 1×10⁴-1×10⁷ cells/ml.

In one embodiment, the preparation may include buffers such as neutral buffered saline, sulfate buffered saline, etc.; carbohydrates such as glucose, mannose, sucrose or dextran, mannitol; proteins; polypeptides or amino acids such as glycine; antioxidants; chelating agents such as EDTA, glutathione; adjuvants (for example, aluminum hydroxide); and preservatives. The preparations of the present invention are preferably formulated for intravenous administration.

### Therapeutic applications

The present invention includes therapeutic applications of the CAR-T cells set forth in the first aspect of the present invention. The transduced T cells can target the tumor cell marker BCMA, synergistically activate T cells, and cause T cell immune responses, thereby significantly improving the killing efficiency against tumor cells.

In light of the foregoing, the present invention further provides a method of stimulating a T cell-mediated immune response to a target cell population or tissue in a mammal. The method includes the steps of: administering the CAR-T cells of the present invention to a mammal.

In one embodiment, the present invention includes a class of cell therapy methods. A patient's autologous T cells (or allogeneic donors) are isolated, activated and genetically modified to produce CAR-T cells, which are then injected into the patient. In this way, the risk of graft-versus-host disease can be extremely low. Antigens are recognized by T cells in an MHC-free manner. In addition, one CAR-T can treat all cancers that express the same antigen. Unlike antibody therapies, CAR-T cells are able to replicate in vivo, resulting in long-term persistence that can lead to sustained tumor control.

In one embodiment, the CAR-T cells of the present invention can undergo robust in vivo T cell expansion for extended amounts of time. In addition, a CAR-mediated immune response can be part of an adoptive immunotherapy procedure in which CAR-modified T cells induce an immune response specific to the antigen binding domain in the CAR. For example, anti-BCMA CAR-T cells can induce specific immune responses against BCMA-expressing cells.

Although the data disclosed herein specifically disclose lentiviral vectors including anti-BCMA scFv, hinge and transmembrane regions, as well as 4-1BB and CD3ζ signaling domains, the present invention should be construed to include any number of variations to each of the components of the construct.

Cancers that can be treated include tumors that are not vascularized or substantially not vascularized, as well as tumors that are vascularized. Cancers may include non-solid tumors (such as hematological tumors, for example, leukemias and lymphomas) or may include solid tumors. The cancer types treated with the CARs of the invention include, but are not limited to, carcinomas, blastomas and sarcomas, and certain leukemia or lymphoid malignancies, benign and malignant tumors, and malignant tumors. Examples are sarcomas, carcinomas and melanomas. The cancers also include adult tumors/cancers and pediatric tumors/cancers.

Hematological cancers can be cancers of the blood or bone marrow. Examples of hematological (or hematogenous) cancers include leukemia, which includes acute leukemias (such as acute lymphoblastic leukemia, acute myeloid leukemia, acute myeloid leukemia and myeloblasts, promyelocytic, myelomonocytic, monocytic, and erythroleukemia), chronic leukemias ( such as chronic myeloid (granulocytic) leukemia, chronic myelogenous leukemia, and chronic lymphocytic leukemia), polycythemia vera, lymphoma, Hodgkin's disease, non-Hodgkin's lymphoma (painless and advanced forms), multiple myeloma, Waldenstrom's macroglobulinemia, heavy chain disease, myelodysplastic syndrome, hairy cell leukemia, and myelodysplasia.

Solid tumors are abnormal masses of tissue that typically do not contain cysts or areas of fluid. Solid tumors can be benign or malignant. Different types of solid tumors are named after the cell type that forms them (such as sarcomas, carcinomas, and lymphomas). Examples of solid tumors such as sarcomas and carcinomas include fibrosarcoma, myxosarcoma, liposarcoma, mesothelioma, lymphoid malignancies, pancreatic cancer, and ovarian cancer.

The CAR-modified T cells of the present invention can also be used as a type of vaccine for ex vivo immunization and/or in vivo therapy of mammals. Preferably, the mammal is a human.

For ex vivo immunization, at least one of the following occurs in vitro prior to administering the cells into a mammal: i) expanding the cells, ii) introducing the CAR-encoding nucleic acid into the cells, and/or iii) cryopreserving the cells.

Ex vivo procedures are well known in the art and will be discussed in detail below. Briefly, cells are isolated from a mammal (preferably human) and genetically modified (for example, transduced or transfected in vitro) with vectors expressing the CARs disclosed herein. CAR-modified cells can be administered to a mammalian recipient to provide therapeutic benefit. The mammalian recipient can be human, and the CAR-modified cells can be autologous to the recipient. Optionally, the cells may be allogeneic, syngeneic, or xenogeneic with respect to the recipient.

In addition to using cell-based vaccines for ex vivo immunization, the present invention also provides compositions and methods for in vivo immunization to elicit an immune response against an antigen in a patient.

The present invention provides methods of treating tumors, which includes administering to a subject in need thereof a therapeutically effective amount of CAR-modified T cells according to the present invention.

The CAR-modified T cells of the present invention can be administered alone or as a pharmaceutical composition in combination with diluents and/or with other components such as IL-2, IL-17 or other cytokines or cell populations. Briefly, the pharmaceutical compositions of the present invention may include a target cell population as described herein in combination with one or more pharmaceutically or physiologically acceptable carriers, diluents or excipients. Such compositions may include a buffer such as neutral buffered saline, sulfate buffered saline, and the like; carbohydrates such as glucose, mannose, sucrose or dextran, mannitol; proteins; polypeptides or amino acids such as glycine; antioxidants; chelating agents such as EDTA or glutathione; adjuvants (for example, aluminum hydroxide); and preservatives. The compositions of the present invention are preferably prepared for intravenous administration.

The pharmaceutical compositions of the present invention may be administered in a manner appropriate to the disease to be treated (or prevented). The amount and frequency of administration can be determined by factors such as a patient's condition, and the type and severity of the patient's disease -- although appropriate doses may be determined by clinical trials.

When referring to an "immunologically effective amount," "anti-tumor effective amount," "tumor-suppressive effective amount," or "therapeutic amount," the precise amount of the composition of the present invention to be administered can be determined by a physician, and it should take into account individual differences in patient's (subject's) age, weight, tumor size, degree of infection or metastasis, and conditions. It may generally be indicated that the pharmaceutical composition including the T cells described herein may be administered at a dose of 10⁴ to 10⁹ cells/kg body weight, preferably 10⁵ to 10⁶ cells/kg body weight (including all integer values within the ranges). The T cell composition can also be administered multiple times at these doses. Cells can be administered by using infusion techniques well known in immunotherapy (reference may be made to, Rosenberg et al., New Eng. J. of Med. 319:1676, 1988). Optimal dosages and treatment regimens for an individual patient can be readily determined by a person skilled in the medical field by monitoring the patient for signs of disease and then adjusting the treatment accordingly.

Administration of a composition to a subject can be carried out in any convenient manner, including nebulization, injection, swallowing, infusion, implantation, and transplantation. The compositions described herein can be administered to a patient subcutaneously, intradermally, intratumorally, intranodally, intraspinally, intramuscularly, intravenous (i.v.) injection, or intraperitoneally. In one embodiment, the T cell composition of the present invention is administered to a patient by intradermal or subcutaneous injection. In another embodiment, the T cell composition of the present invention is preferably administered by i.v. injection. The composition of T cells can be injected directly into tumors, lymph nodes or the site of infection.

In some embodiments of the present invention, the activated and amplified cells can be combined with any number of relevant therapeutic modalities using the methods described herein or other methods known in the art to expand T cells to therapeutic levels (for example, before, concurrently, or after) and administrated to the patient. Such treatment modalities include, but are not limited to, treatment with the following agents: the agents can be antiviral therapy, cidofovir and interleukin-2, cytarabine (also known as ARA-C), natalizumab therapy for MS patients, elfezumab for psoriasis patients, or other treatments for patients with PML. In a further embodiment, the T cells of the present invention can be used in combination with: chemotherapy, radiation, immunosuppressive agents such as cyclosporine, azathioprine, methotrexate, mycophenolate mofetil, and FK506, antibodies or other immunotherapeutic agents. In a further embodiment, the cellular composition of the present invention is administered in combination with (for example, before, concurrently, or after) bone marrow transplantation, chemotherapeutic agents such as fludarabine, external beam radiation therapy (XRT), cyclophosphamide, and then administered to patients. For example, in one embodiment, a subject may undergo standard treatment with high dose chemotherapy, followed by peripheral blood stem cell transplantation. In some embodiments, following the transplantation, the subject receives an infusion of expanded immune cells of the present invention. In an additional embodiment, the amplified cells are administered before or after surgery.

The dose of the above treatments administered to a patient may vary with the precise nature of the condition being treated and the recipient of the treatment. Dose ratios for human administration can be carried out according to art-accepted practice. Typically, 1 × 10⁶ to 1 × 10¹⁰ modified T cells of the present invention (for example, CAR-T20 cells) can be administered to a patient, for example, by intravenous infusion, in each treatment or each course of treatment.

### The main advantages of the present invention include:

(a) The present invention provides Tscm-enriched second generation of universal CAR-T cells, which show better expansion rate, phenotype, IFN-γ releasing ability and killing ability for target cells than the first generation of universal CAR-T cells. Based on the Tscm-enriched T cells of the present invention (especially the second generation of universal CAR-T cells), highly active universal CAR-T cells for different targets can be prepared.
(b) The present invention also prepares universal T cells targeting tumor targets (for example, universal T cells targeting C-CAR088 of BCMA), which can specifically recognize and kill target cells expressing corresponding a tumor target (for example, BCMA tumor antigen).
(c) The present invention provides methods of making Tscm-enriched T cells, and the method can efficiently prepare Tscm-enriched T cell populations, especially universal CAR-T cell populations.

The present invention will be further described below in conjunction with specific embodiments. It should be understood that these embodiments are only used to illustrate the present invention and not to limit the scope of the present invention. For the experimental methods in the following embodiments without specific experimental conditions, conventional experimental conditions are generally followed, for example, as described in Sambrook et al., Molecular Cloning: A Laboratory Manual (New York: Cold Spring Harbor Laboratory Press, 1989), or as suggested by the manufacturers. The percentages and parts used herein are by weight unless otherwise indicated.

### General experimental methods

### 1. Preparation of BCMA-CAR Lentivirus

In the present invention, BCMA is used as a representative tumor-related specific target to prepare CAR-T cells targeting tumor cells.

On day 0, 5 × 10⁶ 293T cells were placed in a 10 cm dish containing 10 ml of DMEM medium pretreated with polylysine. Cells were incubated overnight at 37°C. On day 1, the transfection reagent(s) was prepared. 45.5 µl of lipofectamine 3000 reagent was added to OMEM for a total volume of 500 µl, and then incubated for 5 minutes at room temperature. Another OMEM was prepared with a total volume of 500 µl, and then 10.7 µg BCMA-CAR lentiviral vector, 34 µg PRP2 plasmid, 1.8 µg PRP3 plasmid, 2.3 µg PRM2 plasmid and 36.4 µl P3000 reagent were added. The two reagents were mixed and added into a 1.5 ml tube and incubated for 15 minutes at room temperature. All transfection reagents were added to the 293T cell culture, and the cells were incubated at 37°C for 6 hours. The full medium was discarded and then 10 ml of pre-warmed DMEM medium was added to the cells. The cells were incubated at 37°C for 48 hours. On day 3, the culture supernatant was transferred to a 15 ml tube and centrifuged at 2000 g for 10 minutes at room temperature. Next, every 1 ml of the supernatant was carefully aliquoted into a 1.5 ml tube, and stored at -80°C for later use.

### 2. Stimulation of T cells with anti-CD3 (clone OKT3) antibody (day 3)

500 µl of PBS containing 5 µg/ml fibronectin and 5 µg/ml anti-CD3 antibody was added to wells of a 24 well plate and the plate was incubated at 37°C for 3 hours. The PBS was removed and the wells were washed thoroughly twice with 1 ml of PBS. The plate was ready for T cell stimulation. The number of T cells was counted and 5 × 10⁶ T cells were added to each well, and centrifuged at 100 g for 5 minutes at room temperature. The supernatant was discarded, and the cells were resuspended in 2.5 ml of AIM-V medium and then transferred to a plate coated with an anti-CD3 antibody. The plate was then incubated at 37°C for 72 hours.

### 3. Activation of T cells with CD3/CD28 magnetic beads

T cells were activated with Dynabeads Human T-Activator CD3/CD28 magnetic beads mixed at a ratio of 1:1 with the cells in an AIM-V medium. After 72 hours, the magnetic beads were removed.

### 4. Cell Culture

Tscm cell culture: cell culture was carried out at a density of approximately 1 × 10⁶ cells/ml in an AIM-V medium containing 10% FBS, Glutamax, Pen/Strip, 10 ng/ml IL15, 5 ng/ml IL7, and 10 ng/ml IL21.

Traditional T cell culture: cell culture was carried out at a density of 10⁶ cells/ml in an AIM-V medium supplemented with 10% FBS, Glutamax, Pen/Strip, and 200 IU/ml IL2.

### 5. T Cell Nucleofection (Day 0)

In a PCR tube, 90 pmol of Alt-R CRISPR-Cas9 crRNA antiTRAC (AGA GUC UCU CAG CUG GUA CAG UUU UAG AGC UAU GCU, SEQ ID NO.: 1) was mixed with 90 pmol of Alt-R CRISPR-Cas9 tracRNA (AGC AUA GCA AGU UAA AAU AAG GCU AGU CCG UUA UCA ACU UGA AAA GUG GCA CCG AGU CGG UGC UUU U, SEQ ID NO.: 2).

In another PCR tube, 90 pmol of Alt-R CRISPR-Cas9 crRNA antiB2M (GGC CAC GGA GCG AGA CAU CUG UUU UAG AGC UAU GCU, SEQ ID NO.: 3) was mixed with 90 pmol of Alt-R CRISPR-Cas9 tracRNA.

The above tubes were incubated at 95°C for 5 minutes, and then at 37°C for 25 minutes. After the reaction, the two reagents were mixed well in a test tube. 9 µg of Cas9 protein was added and then incubated at room temperature for 10 minutes. 1 × 10⁶ activated T cells were centrifuged at 100 g for 10 min at room temperature. The supernatant was removed and cells were resuspended in 20 µl of a nucleofection buffer containing 16.4 µl of a P3 buffer and 3.6 µl of a supplemental buffer. The cas9 RNP complex prepared in step 12 was added and then mixed well. The samples were transferred to strips for nucleofection and T cells were transfected with the nucleofection program CA137. After the transfection process was complete, 80 µl of AIM-V medium was added to the strips, then the samples were transferred to the wells in a 24 well plate and an AIM-V medium was added to reach a final total volume of 1 ml. The plate was then incubated at 37°C for 2 hours to prepare for viral transduction.

### 6. BCMA-CAR lentiviral transduction of T cells.

The prepared frozen BCMA-CAR lentiviral supernatant was thawed in a 37°C water bath until it was completely thawed. 1 ml of the BCMA-CAR lentiviral supernatant was transferred to T cell culture to reach a final total volume of 2 ml of the culture medium and protamine sulfate was added to reach a final concentration of 1 µg/ml, which was mixed well by pipetting up and down 5 times. The cells were centrifuged at 2000 g for 2 hours at 32°C, and then the plate was transferred to a 37°C incubator for overnight culture. 1.5 ml of supernatant was carefully taken out on day 1, and then 1.5 ml of fresh AIM-V medium was added. Depending on the cell growth conditions, the medium was changed to maintain a cell density of at least 1 × 10⁶ cells/ml.

### 7. T cell purification (usually on days 6-8)

4 × 10⁶ T cells were transferred to a 1.5 ml tube and centrifuged at 100 g for 10 minutes at room temperature. The supernatant was discarded and the cells were resuspended in 100 µl PBS containing 2% FBS. 2 µl of biotin-conjugated anti-HLA A/B/C and 2 µl of biotin-conjugated anti-CD3 antibody were added to the sample and incubated at 4°C for 30 minutes. 4 µl of biotin isolation mixture was added to the sample and incubated for 15 minutes at room temperature. 5 µl of rapidspheres were added and the sample was incubated at room temperature for 10 minutes. The sample was transferred to a 5 ml tube, and then 1.9 ml of AIM-V medium was added. The tube was placed on the magnet for 5 minutes. The supernatant was transferred to a new tube and placed on the magnet for 5 minutes. The supernatant containing the T cells was transferred to the wells of a 24 well plate.

### 8. FACS analysis

(a). 1×10⁵ T cells were transferred to a 1.5 ml tube and centrifuged at 1500 rpm for 10 minutes at room temperature. The supernatant was discarded, and then 900 µl of PBS was added and then centrifuged at 1500 rpm for 10 minutes at room temperature. The supernatant was discarded and the cells were resuspended in 100 µl of PBS containing 1 µg/ml recombinant human BCMA Fc chimeric protein, and incubated on ice for 1 hour. 900 µl of PBS was added, centrifuged at 1500 rpm for 10 minutes at room temperature, and the supernatant was discarded. The cells were resuspended in PBS (containing 1:1000 Live/Dead aqua orange staining dye), and incubated on ice for 15 minutes. 900 µl of PBS was added, then centrifuged at 1500 rpm for 10 minutes at room temperature, and the supernatant was then discarded. The cells were resuspended in 100 µl of PBS containing 2.5 µl of an APC-Cy7-conjugated anti-CD3 antibody, and 2.5 µl of a V605-conjugated anti-HLA A/B/C antibody and 1; 100 APC-conjugated anti-BCMA Fc chimeric protein antibody in 100 µl of PBS, and then incubated on ice for 30 minutes. 900 µl of PBS was added, centrifuged at 1500 rpm for 10 minutes at room temperature, and the supernatant was discarded. The cells were resuspended in 300 µl of PBS and the sample was transferred to a 5 ml tube for FACS analysis.
   The T-cell populations were firstly grouped by FSC and SSC. The Aqua orange channel was used to differentiate between live and dead cells, and the APC channel was used to differentiate BCMA-CAR positive cells. CD3 and HLA A/B/C were used to differentiate CD3 and HLA A/B/C double knockout T cells.
(b). 1×10⁵ T cells were transferred to a 1.5 ml tube and centrifuged at 1500 rpm for 10 minutes at room temperature. The supernatant was discarded, and then 900 µl of PBS was added and centrifuged at 1500 rpm for 10 minutes at room temperature. The supernatant was discarded and the cells were resuspended in PBS (containing 1:1000 Live/Dead aqua orange staining dye), and incubated on ice for 15 minutes. 900 µl of PBS was added, and centrifuged at 1500 rpm for 10 minutes at room temperature, and the supernatant was discarded. The cells were resuspended in 100 µl of PBS containing 2.5 µl of an APC-conjugated anti-CCR7 antibody, and incubated at 37°C for 30 minutes, followed by the addition of 2.5 µl of a PE-Cy7-conjugated anti-CD45RA antibody, 2.5 µl of a FITC-conjugated Anti-CD8 antibody and 2.5 µl of an APC-Cy7-conjugated anti-CD3 antibody, and then incubated on ice for 30 minutes. 900 µl of PBS was added, centrifuged at 1500 rpm for 10 minutes at room temperature, and the supernatant was discarded. The cells were resuspended in 300 µl of PBS, and the sample was transferred to a 5 ml tube for FACS analysis.

### 9. ELISA

An ELISA plate was coated with Human IFN-γ Mab one day in advance. Human IFN-γ Mab was diluted (1:1000) with 1×PBS to reach 1 µg/mL, 100 µl of the antibody was added to each well, the ELISA plate was sealed with a sealing film, and stored at 4°C overnight. The liquid in the ELISA plate was quickly removed, and then 100 µl of an Assay Buffer was added to each well, sealed with a sealing film, and allowed to stand at room temperature for 30 minutes. The plate was washed: the liquid in the plate was quickly removed, a wash buffer was added with 200 µl per well with a pipette, and the plate was washed again with the foregoing procedure. Human IFN-γ Biotin-labeled Mab was prepared, diluted with the Assay Buffer (1:500), and added to the ELISA plate with 50 µl per well. Human IFN-γ ELISA Standard was also prepared with 8 gradient concentrations (unit pg/ml): 2000, 1000, 500, 250, 125, 62.5, 31.25, and 0.

The sample and the standard were added to the ELISA plate at 100 µl/well. The sample and the standard were then diluted with the Assay Buffer to a desired concentration, sealed with a sealing film, and incubated at room temperature for 1.5 hours. The plate was washed: the liquid in the plate was quickly removed, a wash buffer was added with 200 µl per well with a pipette, and the plate was washed four times. HRP-conjugated streptavidin was prepared, diluted with the Assay Buffer (1:5000), and 100 µl were added per well of the ELISA plate, sealed with the sealing film and incubated at room temperature for 30 minutes. The plate was washed: the liquid in the plate was quickly removed, a wash buffer was added with 200 µl per well with a pipette, and the plate was washed twice. The TMB substrate was warmed to room temperature 30 minutes in advance, and added with 100 µl per well of the ELISA plate. After 5-10 minutes of reaction at room temperature, a stop solution was added with 50 µl/well. The absorbance at 450nm wavelength was detected with a microplate reader. A standard curve was generated according to the concentrations and OD values of the standard, and the IFN-γ concentration of the test sample was calculated according to the OD value of the sample.

### 10. RTCA real-time analysis of cell killing

Day 1: The operating software of RTCA MP was entered and the experimental information was entered. A 96-well E-plate culture plate for RTCA was taken and 50 µl of an F12 complete medium was added to each well of the plate, avoiding air bubbles. The plate was added into the RTCA instrument, and the operating software read the background before adding the cells. It was confirmed that the reading values were normal. Cultured A549 and A549-BCMA cells were trypsinized, and the viable cells were counted using an AO/PI Cell Viability Kit, followed by centrifugation. These two types of tumor cells were resuspended to reach 4 × 10⁵ cells/ml in the F12 complete medium. According to the experimental protocol, 50 µl of the cell suspension (that is, 2 × 10⁴ A549 cells or A549-BCMA cells) was added to each well of the E-plate after reading the background. Three replicate wells were set up in each experimental group of with three wells included as a control group, that is, the tumor cells alone without the addition of T cells subsequently. The plate was placed on a clean bench for 30 minutes to allow cells to adhere evenly. The plate was then placed into the RTCA instrument, and the operating software started to record the changes in the Cell Index. It was set as one reading every 15 minutes, and the total recording duration was set to more than 4 days.

Day 2: The T cells were counted, after centrifugation, the T cells were resuspended in a T cell culture medium and diluted to different concentrations as follows: 1 × 10⁵/ml, 5 × 10⁴/ml, 2.5 × 10⁴/ml, and 1.25 × 10⁴/ml. The RTCA software recording was paused, the E-plate is taken out from the instrument to a clean bench, 100 µl of 1 × 10⁵/ml T cell suspension were added to the culture well of A549 cells, that is, the final ratio of T cells to target cells is 0.5:1; 100 µl of the T cell suspensions of 1 × 10⁵/ml, 5 × 10⁴/ml, 2.5 × 10⁴/ml, 1.25 × 10⁴/ml were added to the culture wells of A549-BCMA cells, that is, the final ratio of T cells to target cells is 0.5:1, 0.25:1, 0.125:1, and 0.0625:1, respectively. The E-plate was put back to the RTCA instrument and the cell index was read every 15 min until the set ending time was reached. After the experiment, data analysis was carried out. In general, the last time point before the addition of T cells was used as a reference, and the read value of tumor cells alone without T cells was set as 100%, and the changes of the tumor cell index after the addition of the T cells were converted into a killing curve.

### Embodiment 1 Analysis of TRAC and B2M gene knockout and purification rates in universal T cells

The knockout of TRAC can result in the disappearance of CD3 expression on the cell surface; the knockout of B2M can result in the disappearance of HLA A/B/C expression on the cell surface. Therefore, gene-edited T cells were stained with anti-HLA A/B/C and anti-CD3 antibodies and flow cytometric analysis was performed to examine the knockout rates of TRAC and B2M.

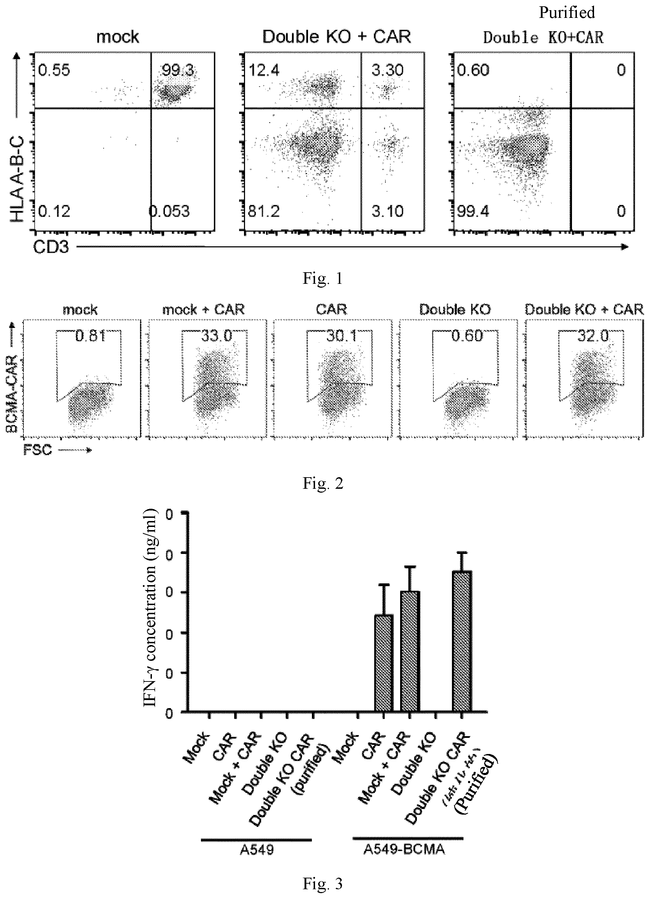

The results are shown in Fig. 1. the double knockout efficiency of TRAC and B2M genes was 81.2% in T cells on day 7 after gene editing with Cas9 RNP and BCMA CAR transduction. After purification, double knockout cells were enriched from 81.4% to 99.4%. However, there were a small number of T cells with low HLA A/B/C expression below the HLA A/B/C gate line.

### Embodiment 2 Efficiency of universal T cell transduction of BCMA CAR

Flow cytometric analysis of T cells transduced with BCMA CAR was performed on day 7.

The results are shown in Fig. 2, the BCMA CAR transduction efficiency was similar between the different groups (mock: T cells without Cas9 RNP nucleofection; mock + CAR: T cells without Cas9 RNP nucleofection but transduced with BCMA CAR; CAR: T cells transduced with BCMA CAR; Double KO: T cells with double knockout of TRAC and B2M after Cas9 RNP nucleofection; Double KO + CAR: T cells obtained by transducing the above Double KO cells with BCMA CAR).

The above results show that the gene editing process of nucleofection does not affect the transduction efficiency of CAR.

### Embodiment 3 Reactivity of universal BCMA CAR-T cells for target cells

The levels of IFN-γ in the supernatants after co-culture of the above-mentioned different BCMA CAR-T cells and the target A549-BCMA cells that express the BCMA antigen were detected by ELISA.

The results are shown in Fig. 3, the reactivity of Double KO + CAR, that is, the gene-edited BCMA CAR-T cells, for the target cells, that is, the ability to release IFN-γ, is similar to those of other unedited conventional BCMA CAR-T cells.

The above results show that the functions of universal CAR-T cells and autologous CAR-T cells are similar.

### Embodiment 4 Preparation of the second generation of universal CAR-T cells

Naive T cells (Tn) were enriched from peripheral blood and flow cytometric analysis was performed to examine the efficiency of enriching CCR7⁺CD45RA⁺ naive T cells (Tn).

### 4.1 Enrichment of Tn cells from peripheral blood

The method is as follows: Human PBMCs were thawed using an AIM-V medium without cytokines, and then the cells were counted. The cells were centrifuged (300 g, 5 min), the supernatant was discarded, and the cells were resuspended in a 5 ml tube with PBS. A biotin isolation cocktail and an anti-TCRγ/δ antibody were added and incubated at room temperature for 5 minutes. The rapidSpheres was added and incubated for 3 minutes at room temperature. A cytokine-free AIM-V medium was added to reach 2.5 ml and the tube was placed on a magnetic stand for 3 minutes. The supernatant was transferred to a new 5 ml tube and placed on the magnetic stand for 3 minutes. The Tn cell-containing supernatant was transferred to a new 15 ml tube, followed by cell counting.

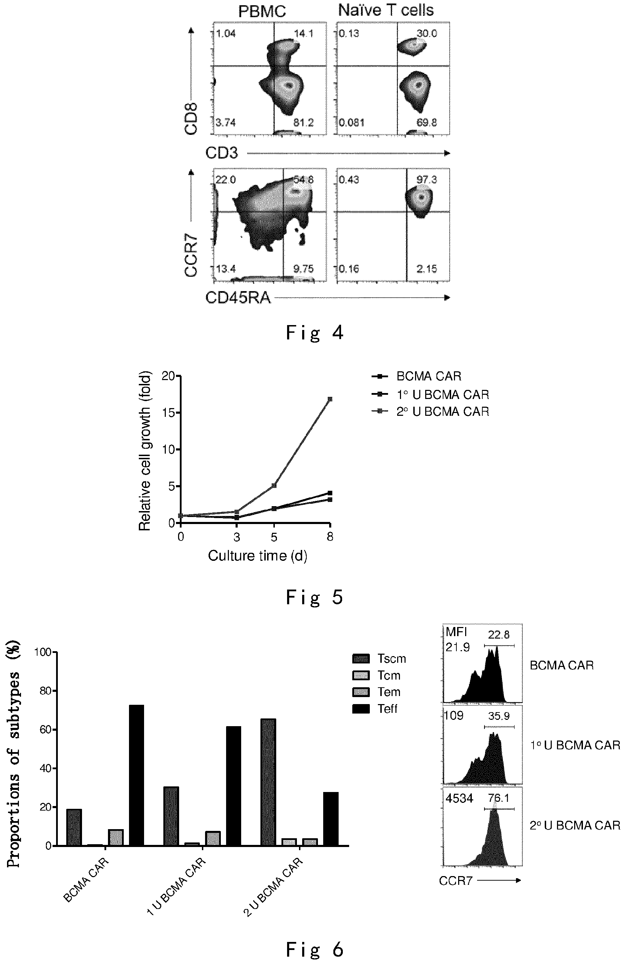

The results are shown in Fig. 4. After enrichment and purification, the proportion of Tn was increased from 54.8% in PBMC to 97.3%. If necessary, the enriched and purified Tn cells can be further expanded and cultured. For example, the expansion can be performed at about 10⁶ cells/ml in an AIM-V medium containing 10% FBS, Glutamax, Pen/Strip, 10 ng/ml IL15, 5 ng/ml IL7 and 10 ng/ml IL21.

### 4.2 Preparation of the second generation of universal CAR-T cells

Embodiments 1-3 were repeated, except that the conventional T cells were replaced by the highly purified Tn cells prepared in 4.1.

For the highly purified Tn cells prepared in 4.1, the TRAC and B2M genes were knocked out, and then the cells were transduced with lentivirus to introduce BCMA CAR so as to prepare the second generation of universal CAR-T cells.

### 4.3 Preparation of the second generation of universal CAR-T cells by direct expansion method (the operation of enriching Tn is omitted)

Another method for preparing the second generation of general-purpose CAR-T cells is as follows:
Starting from the total T cells enriched from peripheral blood (Total T cells), the process of expanding and culturing cells was carried out under suitable conditions (such as in an AIM-V medium containing 10% FBS, Glutamax, Pen/Strip, 10 ng/ml IL15, 5 ng/ml IL7 and 10 ng/ml IL21) at a density of about 10⁶ cells/ml. This resulted in a Tn-enriched population of T cells (under these conditions, Tn cells expand more preferentially and efficiently).

The subsequent gene knockout (such as knockout of TRAC and/or B2M), lentiviral transduction and other steps were further performed to obtain Tscm-enriched universal CAR-T cells, that is, the second generation of universal CAR-T cells.

### Embodiment 5 Expansion efficiency of the second generation of universal T cells

The second generation of universal T cells prepared in Embodiment 4.2 was counted.

The results are shown in Fig.5, the expansion efficiency of the second generation of universal T cells is higher than those of other two types of CAR-T cells. After 7 days, the relative proliferation rate of these cells was as much as 16 times, which was much higher than the 3-4 times obtained from the other two types of CARs. This indicates that the culture conditions for preparing the second generation of universal T cells are more favorable for the proliferation of CAR-T cells than the traditional conditions for preparing CAR.

### Embodiment 6 Proportions of various T cell subtypes in the second generation of universal CAR-T cell product

Flow cytometric phenotype analysis was performed on the second generation of universal CAR-T cell product prepared in Embodiment 4.2.

The results are shown in Fig. 6, the proportion of Tscm in the second generation of universal CAR-T cell product was close to 70%, which is much higher than that in the first generation of universal CAR-T cell product (about 30%) and the autologous CAR-T cells (about 20%).

Unexpectedly, in the second generation of universal CAR-T cells, the percentage of the biomarker CCR7 for de/weakly differentiated cells was as high as 76%. In addition, the MFI value of the second generation of universal CAR-T cells reached 4534, which was much higher than the corresponding MFI values of the first generation of universal CAR-T cell product and the autologous CAR-T cells.

The above results show that the conditions for preparing the second generation of universal CAR-T cells in the present invention not only are conducive to the production of a high proportion of de/weakly differentiated CAR-T cells, that is, Tscm-enriched CAR-T cells, but also allow the produced CAR-T cells to have higher viability and killing activity.

### Embodiment 7 Efficiency of transduction of BCMA CAR in the second generation of universal T cells

The second generation of universal CAR-T cell product prepared in Embodiment 4.2 was subjected to flow analysis of the CAR expression rate.

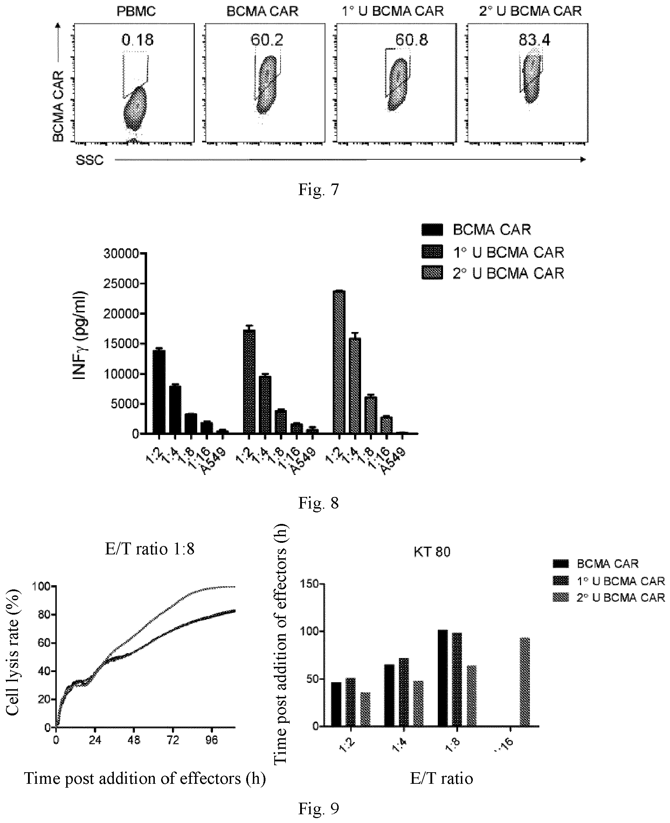

The results are shown in Fig. 7, the proportion of BCMA CAR reached 83.4% in the second generation of universal CAR-T cell product, which was higher than that of the first generation of universal CAR-T cell product (60.8%) and the autologous CAR-T cells (60.2%).

The above results show that the transduction rate of CAR in the second generation of universal CAR-T cells prepared by the present invention does not decrease, but increases.

### Embodiment 8 Reactivity of the second generation of universal BCMA CAR-T cells for target cells

The levels of IFN-γ in the supernatant after co-culture of the above-mentioned different BCMA CAR-T cells and A549-BCMA cells that express BCMA antigen were detected by ELISA.

The results are shown in Fig. 8. The reactivity of the second generation of BCMA CAR-T cells for target cells, that is, the ability to release IFN-γ, was significantly higher than that of the first generation of universal BCMA CAR-T cells and the autologous BCMA CAR-T cells.

In addition, at different effector cell:target cell ratios (from 1:2 to 1:16), the reactivity of the second generation universal CAR-T cells to the target cells is significantly better than those of the other two types of CAR-T cells. This suggests that the second generation of universal CAR-T cells of the present invention has more significant reactivity for the target cells with corresponding target sites (such as tumor antigens, for example, BCMA or other target sites).

### Embodiment 9 Killing ability of the second generation of universal BCMA CAR-T cells for target cells

When the RTCA platform was used, the killing of target cells by the above three BCAM CAR-T cells was monitored for 4 days.

The results are shown in Fig. 9, echoing the results of the above-mentioned IFN-γ release experiments, the second generation of universal BCMA CAR-T cells has the best killing power for target cells.

The cell lysis curve in the left panel of Fig. 9 shows that the second generation of universal BCMA CAR-T cells is more effective at killing target cells than the other two types of CAR-T cells. The result shown herein is an example of a ratio of CAR-T cells to target cells as 1:8. However, the same conclusion applies to other cases of 1:2, 1:4 and 1:16 (data not shown).

The KT80 graph in the right panel of Fig. 9 is the graph of the time required to kill 80% of the target cells. It can be seen that under different ratios of CAR-T cells to target cells, the time required to kill 80% of the target cells by the second generation of universal BCMA CAR-T cells is significantly lower than those of the other two types of CAR-T cells. For example, when the number of CAR-T cells and the number of target cells was 1:8, it took about 70 hours for the second generation of universal cells to kill 80% of the target cells, while it took about 100 hours for the other two types of CAR-T cells to get similar results, which was about 1.5 times longer that of the second generation of CAR-T cells.

In the case where the number of CAR-T cells is much lower than the target cells (1:16), the other two types of CAR-T cells cannot even achieve the object of killing 80% of the target cells, only the second generation of universal BCMA CAR-T Cells can achieve this killing object.

The above results show that the second generation of universal CAR-T cells is more effective in killing target cells than the other two types of CAR-T cells, which is particularly prominent when there are large number of target cells.

### Embodiment 10

In this embodiment, T cells were cultured for expansion under different conditions; except that the added cytokines were changed compared to Embodiment 4, other culture conditions remained the same.

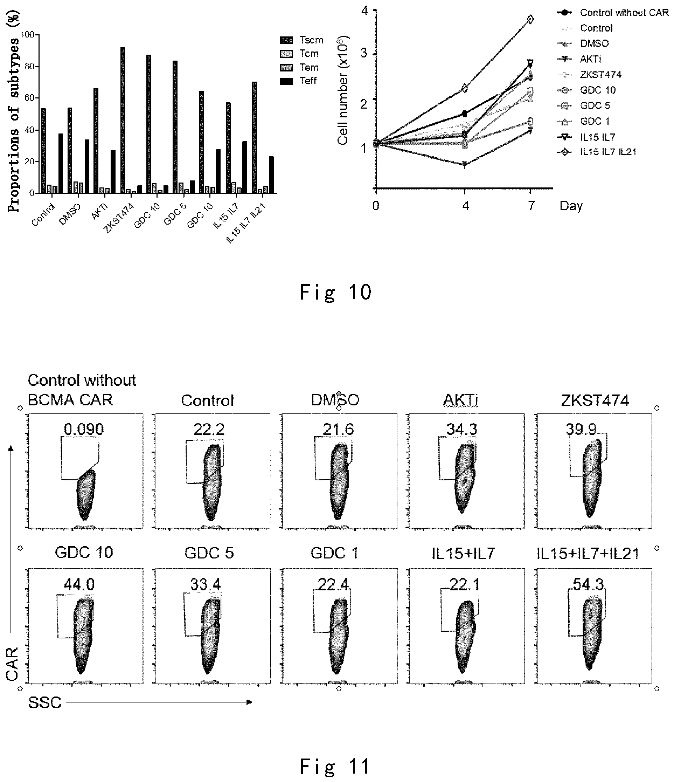

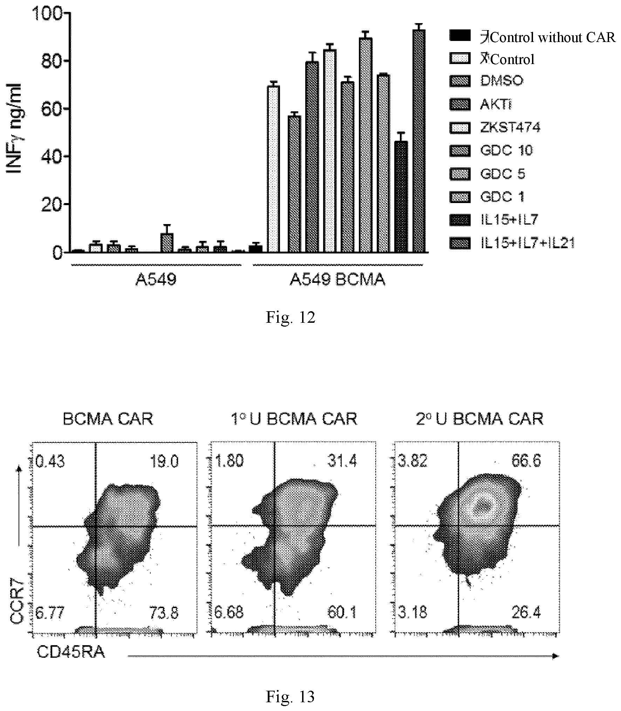

As shown in Figs. 10-13, under different conditions, especially in the presence of different cytokines, T cells were cultured, and the addition of IL1, IL7 and IL21 could significantly increase the enrichment of Tscm cells, promote cell growth, increase BCMA CAR transduction rate, and increase IFN-γ release.

All documents mentioned herein are incorporated by reference in the present application as if each document were individually incorporated herein by reference. In addition, it should be understood that after reading the above teaching contents of the present invention, a person skilled in the art can make various changes or modifications to the present invention. These equivalents also fall within the scope defined by the claims appended hereto.

## Claims

1. A non-naturally occurring T cell population, **characterized in that** a proportion C1 of T memory stem cells (Tscm) in the T cell population is greater than or equal to 50%, calculated by a total number of T cells in the T cell population.

2. The T cell population according to claim 1, **characterized in that** the T cells comprise CAR-T cells.

3. The T cell population according to claim 1, **characterized in that** the T memory stem cells comprise CCR7⁺CD45RA+ T cells.

4. The T cell population according to claim 1, **characterized in that** in the T cell population, a proportion C2 of expressing a biomarker CCR7 is ≥50%, preferably ≥60%, and more preferably ≥70%.

5. The T cell population according to claim 1, **characterized in that** the CAR-T cells are universal CAR-T cells targeting tumor antigens.

6. A cell preparation, **characterized in that** the cell preparation comprises the non-naturally occurring T cell population set forth in claim 1, and a pharmaceutically acceptable carrier, diluent or excipient.

7. A method of preparing CAR-T cells, **characterized in that** the method comprises the following steps:
(a) providing isolated T cells;
(b) performing amplification culture on the isolated T cells in the presence of IL15, IL7 and IL21 to obtain cultured T cells; and
(c) performing engineering modification on the cultured T cells to prepare the CAR-T cells.

8. The method according to claim 1, **characterized in that** in step (a), the isolated T cells are selected from the group consisting of naive T cells (Tn cells), a Tn cell-rich cell population, total T cells, and a combination thereof.

9. The method according to claim 1, **characterized in that** in step (b), when the isolated T cells are cultured for amplification, the concentration of IL15 in the culture system is 1-200 ng/ml, preferably 3-100 ng/ml, and more preferably 5-20 ng/ml; the concentration of IL7 is 0.5-50 ng/ml, preferably 1-20 ng/ml, and more preferably 3-10 ng/ml; and the concentration of IL21 is 1-100 ng/ml, preferably 3-50 ng/ml, and more preferably 5-20ng/ml.

10. A method of preparing CAR-T cells, **characterized in that** the method comprises the following steps:
(i) providing isolated Tn cells;
(ii) optionally, performing amplification culture on the isolated T cells in the presence of IL15, IL7 and IL21 to obtain cultured T cells; and
(iii) modifying the T cells to prepare CAR-T cells.


Sequence Listing

<110>  SHANGHAI CELLULAR BIOPHARMACEUTICAL GROUP LTD.
       WUXI CELLULAR BIOPHARMACEUTICAL GROUP LTD.

<120>  Universal CAR-T cell and preparation and application thereof

<130>  P2020-1051

<150>  CN201910708725.9
<151>  2019-08-01

<160>  3

<170>  PatentIn version 3.5

<210>  1
<211>  36
<212>  RNA
<213>  Artificial sequence

<220>
<223>  crRNA antiTRAC

<400>  1
agagucucuc agcugguaca guuuuagagc uaugcu                              36


<210>  2
<211>  67
<212>  RNA
<213>  Artificial sequence

<220>
<223>  tracRNA

<400>  2
agcauagcaa guuaaaauaa ggcuaguccg uuaucaacuu gaaaaguggc accgagucgg    60

ugcuuuu                                                              67


<210>  3
<211>  36
<212>  RNA
<213>  Artificial sequence

<220>
<223>  crRNA antiB2M

<400>  3
ggccacggag cgagacaucu guuuuagagc uaugcu                              36